# EUROPEAN PATENT SPECIFICATION

(11) **EP 0 456 467 B1**
(45) Date of publication and mention of the grant of the patent: **21.04.1999**
(21) Application number: 91304114.1
(22) Date of filing: 08.05.1991
(51) Int. Cl.: A61L 2/00

(54) **Contact lens cleaning and disinfecting with combinations of polymeric quaternary ammonium compounds and enzymes**
Reinigung und Desinfektion von Kontaktlinsen mittels Kombinationen von polymeren quaternären Ammoniumverbindungen und Enzymen
Nettoyage et désinfection de lentilles de contact par des combinaisons de composés polymériques d'ammonium quaternaire et d'enzymes

(30) Priority: 09.05.1990 US 521218; 25.04.1991 US 689195
(43) Date of publication of application: 13.11.1991
(73) Proprietor: ALCON LABORATORIES, INC., Ft. Worth, TX 76134 (US)
(72) Inventor: Rosenthal, Ruth Ann, Fort Worth, Texas 76134 (US); Van Duzee, Barry F., Willow Park, Texas 76087 (US); Bhatia, Rajkumar, Arlington, Texas 76017 (US)
(74) Representative: Kyle, Diana

(56) References cited:
- EP-A- 0 053 451
- EP-A- 0 384 666
- WO-A-89/11878
- Alcon commercial products descrition OPTI-SOFT nad OPTI-ZYME (revised 1986 print).
- GRAU et al.: "Cleaning Efficacy of Three Disinfection/Enzymatic Cleaning Regimens", PRIMARY EVIDENCE, Allergan Optical Report 001, June 1988, pp. 1-4
- Publication (Sept 1986) relating to Bausch & Lomb ReNu Multi-Action Disinfecting Solution
- CTFA Cosmetic Ingredient Dictionary, published by The Cosmetic Toiletry and Fragance Association, Inc., Washington D.C., p. 245

## Description

The present invention relates to the field of contact lens cleaning and disinfecting. In particular, this invention relates to methods and compositions for the simultaneous cleaning and disinfecting of human-worn contact lenses.

Numerous methods of disinfecting contact lenses have been described. Those methods may be generally characterized as involving the use of heat and/or chemical agents. Representative chemical agents for this purpose include organic antimicrobials such as benzalkonium chloride and chlorhexidine, and inorganic antimicrobials such as hydrogen peroxide and peroxide-generating compounds. U.S. Patents Nos. 4,407,791 and 4,525,346 (Stark) describe the use of polymeric quaternary ammonium compounds to disinfect contact lenses and to preserve contact lens care products. European Patent Application 0 180 309 (Ogunbiyi) describes the use of polymeric biguanides for the same purpose.

There are also numerous known methods of cleaning contact lenses. Some of the more well recognized methods have employed surfactants or enzymes to facilitate cleaning of lenses. The first discussion of the use of proteolytic enzymes to clean contact lenses was in an article by Lot et al. in the Journal of The American Optometric Association, volume 40, pages 1106-1109 (1969). Methods of removing protein deposits from contact lenses by means of proteolytic enzymes have been described in many publications since the initial article by Lo, et al., including U.S. Patent No. 3,910,296 (Karageozian, et al.).

Various methods for cleaning and disinfecting contact lenses at the same time have been proposed. Such methods are described in U.S. Patents Nos. 3,873,696 (Randeri, et al.) and 4,414,127 (Fu), for example. A representative method of simultaneously cleaning and disinfecting contact lenses involving the use of proteolytic enzymes to remove protein deposits and a chemical disinfectant (monomeric quaternary ammonium compounds) is described in Japanese Patent Publication 57-24526 (Boghosian, et al.). Methods involving the combined use of proteolytic enzymes to clean and heat to disinfect are described in European Patent Application 0 141 607 (Ogunbiyi) and PCT Publication No. WO 85/03247 (Perlman). A method involving the combined use of proteolytic enzymes to clean and an inorganic disinfectant (peroxides) to disinfect is described in U.S. Patent No. Re. 32,672 (Huth, et al.).

Grau, et al: "Cleaning Efficacy of Three Disinfection/Enzymatic Cleaning Regimens", PRIMARY EVIDENCE, Allergan Optical Report 001, pages 1-4, June 1988 compares three systems for cleansing and disinfecting contact lenses. The proteolytic activity of the enzymes described in PRIMARY EVIDENCE is not disclosed.

EP A 0 384 666 which is a state of the art document pursuant to Article 54(3)-(4) EPC discloses a method and composition for simultaneously cleaning and disinfecting contact lenses with proteolytic enzymes and antimicrobial agents wherein the lenses are contacted with an aqueous system containing the enzyme and an antimicrobial agent selected from quaternary ammonium salts used in ophthalmic applications and biguanides and wherein the osmotic value of the system is maintained at a level which does not substantially inhibit the activity of the antimicrobial agent. The system can include a chelating agent such as ethylenediaminetetraacetic acid. The proteolytic activity of the enzymes used in the system of EPA 0 384 666 is not disclosed.

### Summary of the Invention

The present invention is directed to an improved method for cleaning and disinfecting contact lenses at the same Time. More particularly, this invention involves the combined use of enzymes to clean and a very low concentration of a polymeric antimicrobial agent to disinfect. The present invention is based on the finding that these antimicrobial agents can be effectively utilized in combination with enzymes to clean and disinfect contact lenses under certain conditions. This finding is surprising, both in view of prior art attempts which indicate that there is mutual inhibition, possibly by binding between the enzyme and the antimicrobial agent, and in light of other, earlier observations that large excesses of one or both of the enzyme and antimicrobial agent were required to achieve both cleaning and disinfecting.

The present invention is based in part on the finding that the use of certain formulation criteria can eliminate or reduce the above-cited binding problem. More particularly, it has been found that this binding can be substantially eliminated by using certain "complexing agents" (defined hereunder) to prevent binding between the enzyme and antimicrobial agent, thereby maintaining the activity of each. The problem can be further overcome by maintaining the tonicity of the solution containing the antimicrobial agent at an isotonic or hypotonic level to maximize antimicrobial activity and by controlling both the enzyme and antimicrobial agent concentrations. This approach allows a very low concentration of the antimicrobial agent to be utilized, while still achieving both disinfecting and cleaning of the lenses.

### Brief Description of the Drawing

Each of Figures 1 through 6 is a graphic illustration of log₁₀ reduction of a microorganism over exposure time in the disinfecting solution control ("diamond" points) and in the combination enzymatic cleaner and disinfecting solution ("+" points).
Figure 1 illustrates log₁₀ survivors of S. epidermidis over exposure time (hr).
Figure 2 illustrates log₁₀ survivors of P. aeruginosa over exposure time (hr).
Figure 3 illustrates log₁₀ survivors of S. marcescens over exposure time (hr).
Figure 4 illustrates log₁₀ survivors of C. albicans over exposure time (hr).
Figure 5 illustrates log₁₀ survivors of A. fumigatus over exposure time (hr).
Figure 6 illustrates log₁₀ survivors of Herpes simplex over exposure time (min).

### Detailed Description of the Invention

The enzymes which may be utilized in the compositions of the present invention include all enzymes which: (1) are useful in removing deposits from contact lenses; (2) cause, at most, only minor ocular irritation in the event a small amount of enzyme contacts the eye as a result of inadequate rinsing of a contact lens; (3) are chemically stable and effective in the presence of the polymeric antimicrobial agents described below; and (4) do not adversely affect the physical or chemical properties of the lens being treated. For purposes of the present specification, enzymes which satisfy the foregoing requirements are referred to as being "ophthalmically acceptable."

With the foregoing requirements as a guide, those skilled in the art of contact lens care will readily appreciate that numerous enzymes may be utilized in the present invention. Since it is conventional in this art to categorize enzymes according to their activities, it can be further stated that the enzymes useful in the present invention include those having proteolytic, lipolytic, mucolytic, and/or amylolytic activity. Enzymes having proteolytic activity are particularly important, since protein deposits on contact lenses are a common problem. Reference is made to the above-cited U.S. Patents Nos. 3,910,296 and Re. 32,672 for a detailed listing of enzymes which are generally recognized as being useful for cleaning contact lenses.

The enzymes utilized in the present invention will most frequently be enzymes having proteolytic, lipolytic, and/or amylolytic activity since deposits of proteins, lipids and mucopolysaccharides are formed on most, if not all, human-worn contact lenses. Examples of suitable proteolytic enzymes include: pancreatin, subtilisin, papain, ficin, and bromelin. Examples of suitable lipolytic enzymes include: Lipase K-3000 and Lipase AP6 (available from Amano Corp., Troy, VA), and pancreatic lipase. Examples of suitable amylolytic enzymes include: α-amylase, β-amylase, aminoglucosidase (glucoamylase), fungal amylase, and pullalanase. Such enzymes are commercially available from various sources, including Novo Industries (Bagsvaerd, Denmark), Sigma (St. Louis, MO), and Boehringer Mannheim (Indianapolis, IN). Those skilled in the art will readily appreciate that other enzymes of plant, animal, bacterial, fungal, or synthetic origin might also be employed in the present invention. Certain enzymes may require the presence of a stabilizing agent, such as acetylcysteine.

The methods of the present invention will typically involve the use of an amount of one or more enzymes effective to remove substantially or to reduce significantly deposits of proteins, lipids, mucopolysaccharides and other materials on the lenses. For purposes of the present specification, such an amount is referred to as "an amount effective to clean the lens." The amount of enzyme or enzymes utilized in particular embodiments of the present invention may vary, depending on various factors, such as the type of enzyme(s) utilized, the proposed duration of exposure of lenses to enzyme, the nature of the lens care regimen (e.g., the frequency of lens disinfection and cleaning), the type of lens being treated, and the use of adjunctive cleaning agents (e.g., surfactants). In general, one or more enzymes in an amount less than or equal to about 50 proteolytic activity units per milliliter (ml) of solution will be utilized, preferably less than about 26 proteolytic activity units, and most preferably less than about 13 proteolytic activity units. For purposes of this specification, a "proteolytic activity unit" is defined as the amount of enzyme activity present to generate one microgram (µg) of tyrosine per minute (µg Tyr/min), as determined by the casein-digestion, colorimetric assay described below.

### Casein-digestion assay

A 5.0 ml portion of casein substrate (0.65% casein w/v) is equilibrated for 10 minutes (min) ± 5 seconds (s). A 1.0 ml portion of enzyme solution (0.2 mg/ml) is then added to the casein substrate and the mixture vortexed, then incubated for 10 min ± 5 s. After incubation, 5.0 ml of 14% trichloroacetic acid is added and the resultant mixture immediately vortexed. The mixture is incubated for at least another 30 min, then vortexed and centrifuged for 15-20 min (approx. 2000 rpm). The supernatant of the centrifuged sample is filtered into a serum filter sampler and a 2.0 ml aliquot removed. To the 2.0 ml sample is added 5.0 ml of 5.3% Na₂CO₃. The sample is vortexed, 1.0 ml of 0.67N Folin's Phenol reagent is added, and the sample is immediately vortexed again, then incubated for 30 min at 37°C. The sample is then read on a visible light spectrophotometer at 660 nanometers (nm) versus purified water as the reference. The sample concentration is then determined by comparison to a tyrosine standard curve.

Enzymes having lipolytic, amylolytic, and/or mucolytic activity will typically be utilized in amounts of less than or equal to about 5 United States Pharmacopeia (USP) units per ml of solution.

As used in the present specification, the term "polymeric antimicrobial agent" or "antimicrobial agent" refers to any nitrogen-containing polymer or co-polymer which has antimicrobial activity. Suitable antimicrobial agents include, but are not limited to: polymeric quaternary ammonium compounds, such as disclosed in U.S. Patents Nos. 3,931,319 (Green, et al.), 4,026,945 (Green, et al.) and 4,615,882 (Stockel, et al.); and polymeric biguanides, such as disclosed in U.S. Patent No. 4,537,746 (Ogunbiyi, et al.) and European Patent Application 0 180 309 (Ogunbiyi). Particularly preferred are:
(I) polymeric quaternary ammonium compounds of structure: 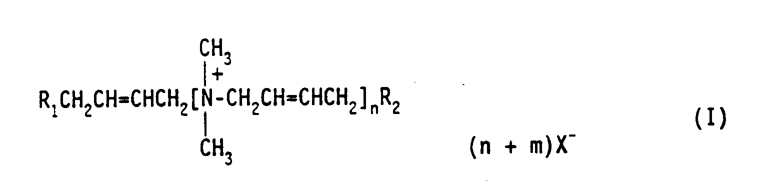 wherein: R₁, R₂ = OH, N(CH₃)₂, or N⁺(CH₂CH₂OH)₃; X is a pharmaceutically acceptable anion, preferably chloride; m = 0, 1 or 2; and the average molecular weight is generally in the range of about 2,000 to 30,000, preferably in the range of about 3,000 to about 14,000; or mixtures thereof, and
(II) polymeric biguanides of structure: 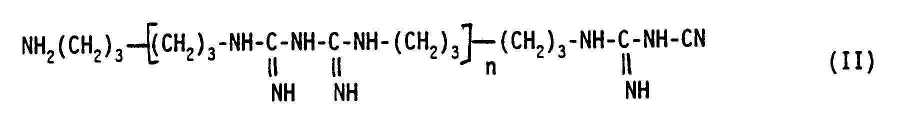 or pharmaceutically acceptable salts thereof, wherein the average molecular weight is generally in the range of about 500 to about 120,000 and preferably in the range of about 500 to about 2,500. The use of these particularly preferred antimicrobial agents to disinfect are described in U.S. Patents Nos. 4,407,791 (Stark) and 4,525,346 (Stark), and European Patent Application 0 180 309 (Ogunbiyi), respectively.

The above-described antimicrobial agents are utilized in the methods of the present invention in an amount effective to eliminate substantially or to reduce significantly the number of viable microorganisms found on contact lenses, in accordance with the requirements of governmental regulatory agencies, such as the United States Food and Drug Administration (e.g., 1985 Guidelines). For purposes of the present specification, that amount is referred to as being "an amount effective to disinfect" or "an antimicrobial effective amount." The amount of antimicrobial agent employed will vary, depending on factors such as the type of lens care regimen in which the method is being utilized. For example, the use of an efficacious daily cleaner in the lens care regimen may substantially reduce the amount of material deposited on the lenses, including microorganisms, and thereby lessen the amount of antimicrobial agent required to disinfect the lenses. The type of lens being treated (e.g., "hard" versus "soft" lenses) may also be a factor. In general, a concentration in the range of 0.000001% to 0.01% by weight of one or more of the above-described antimicrobial agents will be employed. The most preferred concentration of the polymeric quaternary ammonium compounds of Formula (I) is about 0.001% by weight, whereas the most preferred concentration of the polymeric biguanides of Formula (II) is about 0.00005% by weight.

As explained above, it has been found that even at very low concentrations, the above-described antimicrobial agents can be effectively utilized in combination with one or more enzymes to clean and disinfect contact lenses at the same time, provided that certain conditions are maintained. More specifically, it has been found that certain low molecular weight compounds having at least one carboxy functional group can be utilized to reduce substantially binding between the positively charged antimicrobial agents and the negatively charged sites of the enzymes. Although Applicants do not wish to be bound to a particular theory, it is believed that the complexing agent substantially reduces binding between enzyme and antimicrobial agent by binding with the antimicrobial agent, thereby effectively blocking the positively charged sites on the antimicrobial agent from binding or complexing with the negatively charged sites on the enzyme. Thus, a very low concentration of the antimicrobial agent can be utilized because the enzyme is effectively prevented from disrupting the disinfecting activity of the antimicrobial agent. For purposes of the present specification, these low molecular weight compounds having at least one carboxy functional group are referred to as "complexing agents." The complexing agents significantly improve the performance of the antimicrobial agents without adversely affecting the performance of the enzymes contained in the same solutions. By keeping the ionic strength of the solution at an isotonic or hypotonic level and by limiting the enzyme concentration, the activity of the antimicrobial agent is further enhanced.

The complexing agents which may be utilized include, but are not limited to: ethylenediamine-tetraacetic acid, citric acid, acetic acid and their pharmaceutically acceptable salts. The amount of complexing agent utilized will depend on several factors, including the particular type of agent selected and the relative concentrations of enzyme and antimicrobial agent utilized. The concentration will typically range from about 0.03% to about 0.7% by weight/volume, with 0.05% to 0.6% preferred.

The ionic strength or tonicity of the cleaning and disinfecting solution of the present invention has also been found to be an important factor. More specifically, it has been found that polymeric ammonium compounds, and particularly those of Formula (I), above, lose antimicrobial activity as the tonicity of the solution is increased. A preferred tonicity in the range of 150 to 300 milliOsmoles per kilogram (mOs/kg) is therefore utilized, with a range of 210 to 240 mOs/kg being more preferred and a tonicity of 220 mOs/kg being most preferred. The tonicity of the solution may be affected by various components of the solution, but it is generally a function of the sodium chloride concentration.

Other factors have also been found to have a significant influence on the combined use of enzymes and antimicrobial agents in accordance with the present invention. In particular, it has been found that, in combining one or more enzymes, the contact lens to be treated, and the antimicrobial agent in an appropriate solution (e.g., saline or distilled water), the sequence of release into solution is important. In order to achieve maximum killing of microorganisms, the lens and the antimicrobial agent should be combined in solution before the enzyme is released into the solution. Thus, if the enzyme is formed as a conventional tablet (or as a separate solution), the enzyme tablet (or solution) should be added after the lens and the antimicrobial agent have been placed together in solution; but if the enzyme is formed as a tablet having a delayed release coating, the enzyme tablet may be added to the solution at any point in the sequence of addition, provided that the actual release of enzyme into solution occurs after the lens and the antimicrobial agent have been combined in the solution.

In addition, the antimicrobial activity of the enzyme and antimicrobial agent combination has surprisingly been found to become even more effective than the antimicrobial agent alone when lenses are treated for extended periods of approximately one hour to overnight, with four to eight hours preferred. Since, for the sake of convenience, contact lenses are typically soaked overnight in order to be cleaned with enzymes or disinfected with chemical agents, this finding has practical significance.

The contact lens cleaning and disinfecting system of the present invention may be provided in various physical forms. For example, the two main components, antimicrobial agent and enzyme, can be formulated separately, or a single dosage form may be used (see Example 2). If formulated separately, the antimicrobial agent is generally provided in solution form, although it could also be provided in solid form. The enzyme cleaner can be provided in either solid or liquid form, but for packaging and dosage purposes, effervescent compositions in tablet form are desirable. Dosage forms which provide for delayed release of the enzyme in solution are particularly preferred. If formulated together, the two components may be formed in layers with the enzyme component being formed as a core tablet and the antimicrobial agent being formed as an outer layer around said enzyme core, with a seal and/or a delayed release coating interposed between the enzyme core and the antimicrobial agent outer layer.

In the above-described embodiments of the invention wherein the enzyme is formulated as a delayed release tablet or the enzyme and antimicrobial agent are combined in a single tablet, the delayed release coating can be composed of any of several polymeric materials, including cellulose ethers, vinyls, glycols, and acrylics. The delayed release coating may additionally include plasticizers such as propylene glycol, polyethylene glycol, glycerin, mineral oil, vegetable oil, or other known plasticizers. The seal coating may be composed of any of several polymeric materials, including cellulose ethers, vinyls, glycols, and acrylics, or the coating could be composed of synthetic or natural gums, gelatin, shellac, salts (e.g., sodium chloride), saccharide alcohols (e.g., mannitol or sorbitol), or other commonly used seal coating materials.

The method of use of this invention will depend on the dosage form employed, but, in general, the procedure of Example 4 will be followed.

The following examples are presented to illustrate further various aspects of the present invention, but are not intended to limit the scope of the invention in any respect.

### EXAMPLE 1

A formulation in which the antimicrobial agent and enzyme cleaner components are provided separately is illustrated below.

### A. Disinfecting Solution

The following formulation represents a preferred disinfecting solution:

| Ingredient | (w/v)% |
|---|---|
| Polyquad® | 0.001 + 10% excess |
| Sodium chloride | 0.48 |
| Disodium edetate | 0.05 |
| Citric acid monohydrate | 0.021 |
| Sodium citrate dihydrate | 0.56 |
| Purified Water | q.s. |

| | |
|---|---|
| *Polyquad® is a chloride salt of the polymeric quaternary ammonium compound of Formula (I) having an average number molecular weight of 5000 - 7000. The chemical name of this salt is: α-4-[1-tris(2-hydroxyethyl)ammonium-2-butenyl] poly[1-dimethy]ammonium-2-butenyl]-ω-tris(2-hydroxyethyl)ammonium chloride. | |

To prepare the above formulation, sodium citrate dihydrate, citric acid monohydrate, disodium edetate, sodium chloride and Polyquad®, in the relative concentrations indicated above, were mixed with purified water and the components allowed to dissolve by stirring with a mixer. Purified water was added to bring the solution to almost 100%. The pH was recorded at 6.3 and adjusted to 7.0 with NaOH. Purified water was added to bring the solution to 100%. The solution was stirred and a pH reading of 7.0 was taken. The solution was then filtered into sterile bottles and capped.

### B. Enzymatic Cleaner Tablet

The following composition represents a preferred enzymatic cleaner tablet:

| Ingredient | mg/50 mg Tablet |
|---|---|
| Pancreatin (6X) | 1.38* |
| Citric Acid | 5.95 |
| Sodium Bicarbonate | 13.135 |
| Povidone (K 29-32) | 0.415 |
| Polyethylene Glycol (3350) | 0.75 |
| Compressible Sugar | QS |
| Alcohol | QS** |

| | |
|---|---|
| *corresponds to 105.5 + 10% excess mcg Tyrosine/min | |
| **evaporated during processing | |

The above ingredients are combined and formed into tablets of appropriate size and hardness, according to methods known to those skilled in the art.

This tablet may also be formulated with a seal coating and/or a delayed release coating to provide for a delay in dissolution of up to about 2 hours.

### EXAMPLE 2

A single dosage form which contains both the antimicrobial agent and enzyme is illustrated below.

| Ingredient | Quantity |
|---|---|
| Enzymatic Cleaner Core | (mg/Tablet) |
| Pancreatin (6X) | 1.38* |
| Citric Acid (Anhydrous) | 4.0 |
| Sodium Bicarbonate | 8.5 |
| Polyethylene Glycol (3350 Powder) | 2.0 |
| Lactose | 42.5 |

| Seal Coat | (Percent by Weight) |
|---|---|
| Hydroxy Methyl Cellulose | 5.0 |
| Ethanol (USP. Dehydrated) | 75.8 |
| Purified Water | 19.2 |

| Delayed Release Coat | (Percent by Weight) |
|---|---|
| Acrylic Acid Ester (30% Solids w/w/) | 29.7 |
| Polyethylene Glycol (8,000) | 1.0 |
| Purified Water | 69.3 |

| Disinfectant Coat | (Percent by Weight) |
|---|---|
| Polyquad® | 0.16 |
| Sodium Chloride | 4.8 |
| Hydroxypropyl Methyl Cellulose (2910, E-5 Premium) | 0.8 |
| Purified Water | 94.24 |

| | |
|---|---|
| *Corresponds to 105.5 + 10% excess mcg Tyrosine/min | |

To prepare the enzymatic cleaner core, pancreatin, polyethylene glycol, and lactose, in the amounts indicated above, are mixed in a blender for 5 min, citric acid is added and the composite mixed for another 5 min, at which time sodium bicarbonate is added and the composite mixed for another 20 min. The resultant granulation is then compressed using a rotary tablet press fitted with suitable tooling into tablets of appropriate size and having a hardness of 5 to 10 Strong-Cobb units. The tablets are then sequentially coated with a seal coat (2.5% tablet weight gain), a delayed release coat (17.5% tablet weight gain), and a disinfectant coat (3.6% tablet weight gain). Each of the coating solutions is similarly prepared by mixing the ingredients until a homogenous mixture is obtained. Each of the coating solutions is applied by rotating the tablets and spraying the coating solution onto the tablets. After each coating solution is applied, the tablets are allowed to dry before the next coating solution is applied.

All portions of tablet preparation should be done under low humidity conditions (below 20% relative humidity).

### EXAMPLE 3

The disinfecting effectiveness of the present invention was evaluated by determining the rate and extent of kill for six logarithmic challenge innoculae. The invention was tested against six microorganisms: Staphylococcus epidermidis, Pseudomonas aeruginosa, Serratia marcescens, Candida albicans, Aspergillus fumigatus and Herpes simplex.

A 0.1 ml volume of inoculum was first added to a 10 ml volume of the disinfecting solution of Example 1, followed by the addition of one 50 mg tablet of the enzymatic cleaner of Example 1. A similarly inoculated 10 ml volume of the disinfecting solution of Example 1 was used as a control. The solutions were maintained at room temperature throughout the test. Each microorganism and test solution was tested individually. Sets of four replicate (n = 4) samples were tested for each organism, except for Herpes simplex, where only two samples were tested (n = 2).

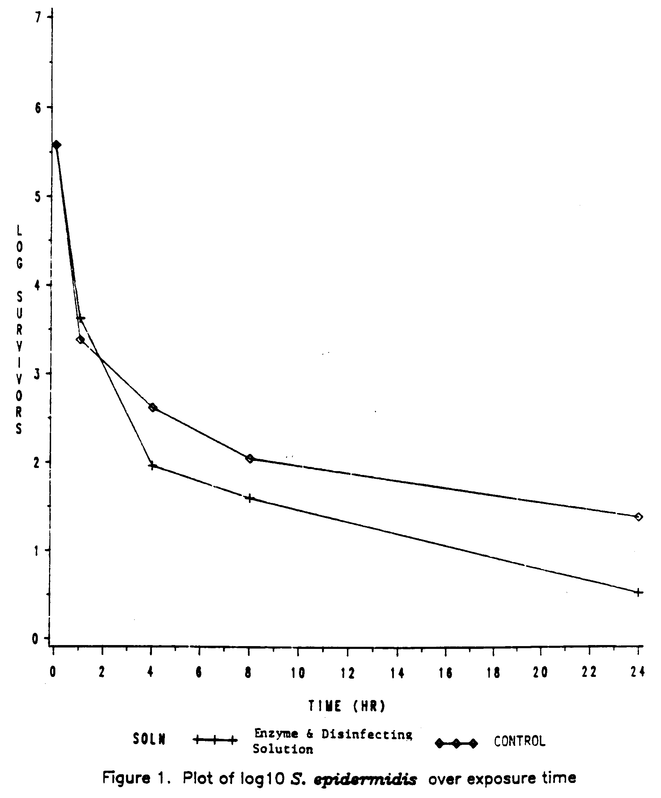

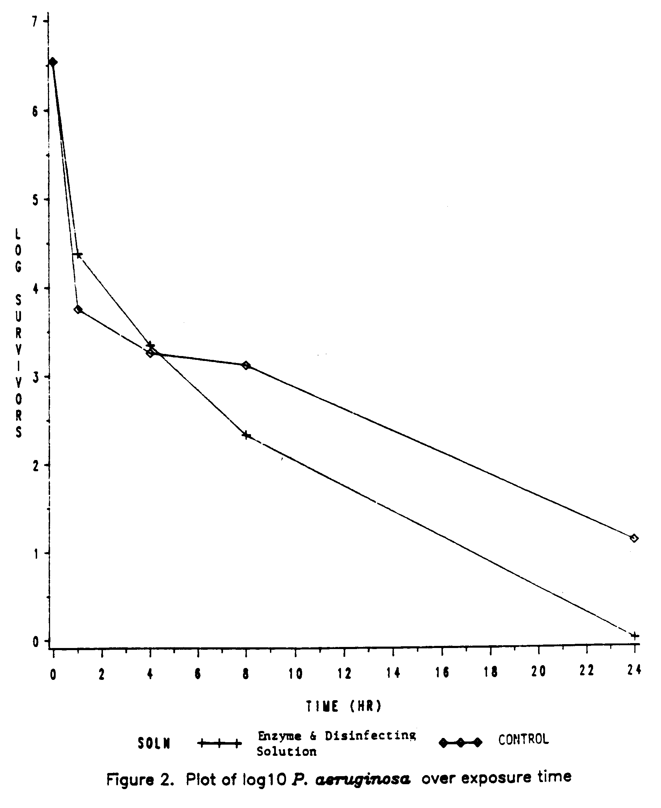

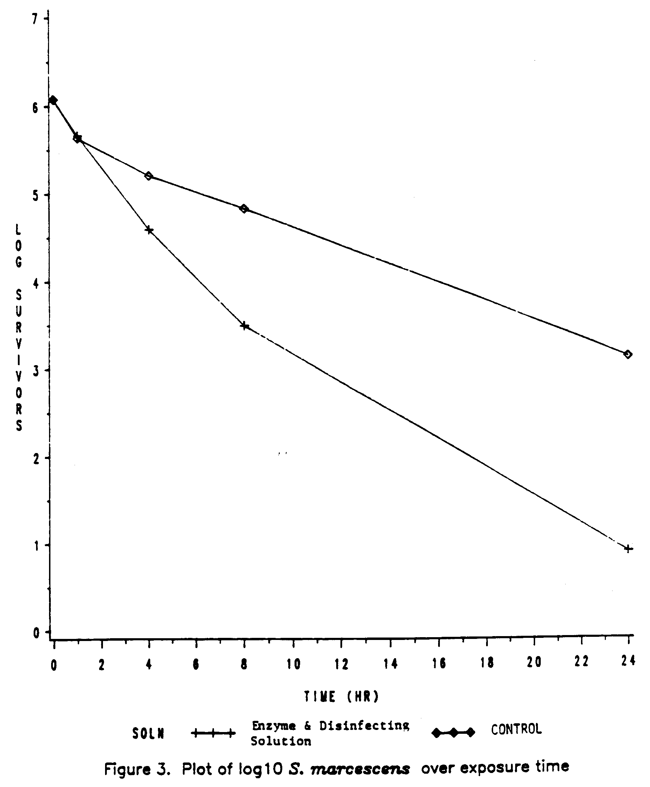

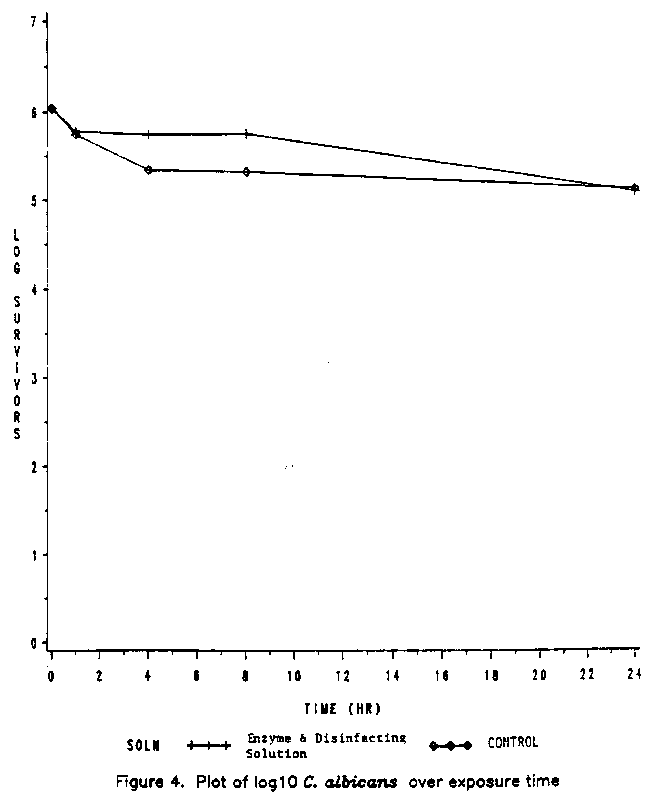

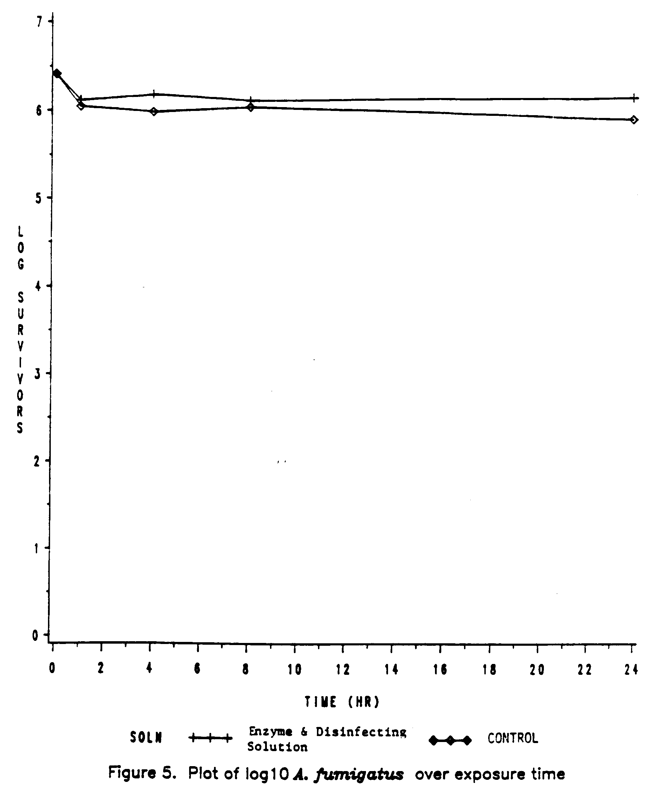

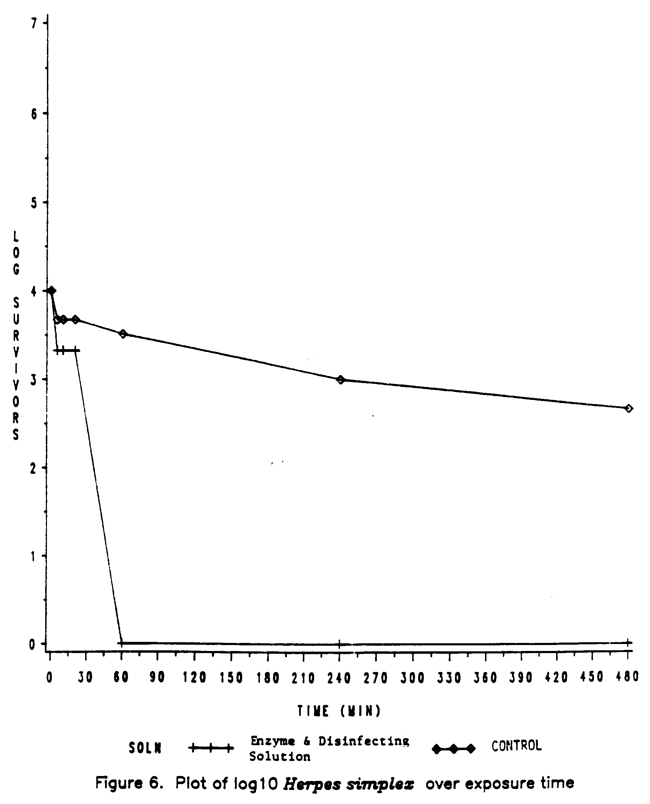

At selected time intervals of 0, 1, 4, 8 and 24 hours, a 1 ml volume of the inoculated test solution was removed and appropriate serial dilutions were made in sterile 0.9% NaCl dilution blanks. Pour-plates were prepared with soybean-casein digest agar containing 0.07% Asolectin and 0.5% Polysorbate 80. At Time 0, a 1.0 ml volume of the saline control was removed and serial dilution pour-plates were prepared using the same recovery medium and dilution blanks. The Time 0 saline control count was used as the initial count. The pour-plates were incubated at 30°-35°C for appropriate incubation periods. The number of surviving organisms at each time interval was then determined. The D-value was calculated in hours by the Stumbo method (Stumbo, C.R., "A Technique for Studying Resistance of Bacterial Spores to Temperature in the Higher Range." Food Technology 2:228, 1948). The results are summarized in Tables I - VI below and plotted in Figures 1-6.

**TABLE I**

| ANTIMICROBIAL ACTIVITY S. EPIDERMIDIS | | |
|---|---|---|
| SAMPLE | TIME(h) | LOG REDUCTION |
| Enzymatic Cleaner in disinfecting solution | 1 | 2.0 |
| | 4 | 3.6 |
| | 8 | 4.0 |
| | 24 | 5.1 |
| Disinfecting solution control | 1 | 2.2 |
| | 4 | 3.0 |
| | 8 | 3.5 |
| | 24 | 4.2 |

**TABLE II**

| ANTIMICROBIAL ACTIVITY AGAINST P. AERUGINOSA | | |
|---|---|---|
| SAMPLE | TIME(h) | LOG REDUCTION |
| Enzymatic cleaner in disinfecting solution | 1 | 2.2 |
| | 4 | 3.2 |
| | 8 | 4.2 |
| | 24 | 6.5 |
| Disinfecting solution control | 1 | 2.8 |
| | 4 | 3.3 |
| | 8 | 3.4 |
| | 24 | 5.4 |

**TABLE III**

| ANTIMICROBIAL ACTIVITY AGAINST S. MARCESCENS | | |
|---|---|---|
| SAMPLE | TIME(h) | LOG REDUCTION |
| Enzymatic cleaner in disinfecting solution | 1 | 0.4 |
| | 4 | 1.5 |
| | 8 | 2.6 |
| | 24 | 5.2 |
| Disinfecting solution control | 1 | 0.4 |
| | 4 | 0.9 |
| | 8 | 1.3 |
| | 24 | 2.9 |

**TABLE IV**

| ANTIMICROBIAL ACTIVITY OF COMBINATION AGAINST C. ALBICANS | | |
|---|---|---|
| SAMPLE | TIME(h) | LOG REDUCTION |
| Enzymatic cleaner in disinfecting solution | 1 | 0.3 |
| | 4 | 0.3 |
| | 8 | 0.3 |
| | 24 | 0.9 |
| Disinfecting solution control | 1 | 0.3 |
| | 4 | 0.7 |
| | 8 | 0.7 |
| | 24 | 0.9 |

**TABLE V**

| ANTIMICROBIAL ACTIVITY OF COMBINATION AGAINST A. FUMIGATUS | | |
|---|---|---|
| SAMPLE | TIME(h) | LOG REDUCTION |
| Enzymatic cleaner in disinfecting solution | 1 | 0.3 |
| | 4 | 0.2 |
| | 8 | 0.3 |
| | 24 | 0.2 |
| Disinfecting solution control | 1 | 0.4 |
| | 4 | 0.4 |
| | 8 | 0.4 |
| | 24 | 0.5 |

**TABLE VI**

| ANTIMICROBIAL ACTIVITY OF COMBINATION AGAINST HERPES SIMPLEX | | |
|---|---|---|
| SAMPLE | TIME(MINS) | LOG REDUCTION |
| Enzymatic cleaner in disinfecting solution | 5 | 0.7 |
| | 10 | 0.7 |
| | 20 | 0.7 |
| | 60 | 4.0 |
| | 240 | 4.0 |
| | 480 | 4.0 |
| Disinfecting solution control | 5 | 0.3 |
| | 10 | 0.3 |
| | 20 | 0.3 |
| | 60 | 0.5 |
| | 240 | 1.0 |
| | 480 | 1.3 |

The average D-values (see Table VII, below) for the enzymatic cleaner and disinfecting solution combination and the disinfecting solution control were less than or equal to 0.5 h for S. epidermidis and P. aeruginosa, less than or equal to 2.5 h for S. marcescens, less than or equal to 3.9 hr for C. albicans, and less than or equal to 3.4 h for A. fumigatus. The average D-values for the enzymatic cleaner and disinfecting solution combination were less than a quarter of the average D-values of the disinfecting solution control for Herpes simplex.

No statistically significant difference in D-values between the enzymatic cleaner and disinfecting solution combination and the disinfecting solution control was found for S. epidermidis, S. marcescens, C. albicans, and A. fumigatus. A small statistically significant difference (0.1 h) in D-values between the combination and the disinfecting solution control was calculated against P. aeruginosa (Table II); however, the difference was not considered to be microbiologically significant. Moreover, the combination showed more rapid overall bactericidal and virucidal activity than the disinfecting solution control.

**TABLE VII**

| D-VALUE SUMMARY | | | | |
|---|---|---|---|---|
| Organism | Average D-Value (h) ± SD^{a} | | | |
| | Enzymatic cleaner in disinfecting solution | Control | Analysis | |
| S. epidermidis | 0.5 ± 0.1 | 0.5 ± 0.1 | t=1.24 | (6.0)^{b} |
| | n=4^{c} | n=4 | p=0.13 | |
| P. aeruginosa | 0.5 ± 0.0^{d} | 0.4 ± 0.0 | t=4.35 | (6.0) |
| | n=4 | n=4 | p=0.00 | |
| S. marcescens | 2.5 ± 0.7 | 2.3 ± 0.7 | t=0.28 | (6.0) |
| | n=4 | n=4 | p=0.40 | |
| C. albicans | 3.9 ± 0.3 | 3.4 ± 0.6 | t=1.59 | (6.0) |
| | n=4 | n=4 | p=0.08 | |
| A. fumigatas | 3.4 ± 1.1 | 2.6 ± 0.4 | t=1.32 | (6.0) |
| | n=4 | n=4 | p=0.12 | |
| Herpes simplex | 0.17, 0.38 | 2.9, 2.0 | | |
| | n=2 | n=2 | | |

| | | | | |
|---|---|---|---|---|
| ^{a}Standard deviation | | | | |
| ^{b}Degrees of freedom | | | | |
| ^{c}Number of tests | | | | |
| ^{d}Values of 0.00 are due to round-off | | | | |

Of greater statistical significance than the average D-values were the differences in log reduction over time between the enzymatic cleaner an disinfecting solution combination and the disinfecting solution control. Comparison of actual log reduction, using a single, specified time for kill of microorganisms, is a more accurate determination of antimicrobial activity than comparison of D-values, since D-values are calculated from the initial log reduction and do not therefore take into consideration any subsequent reduction.

As is graphically illustrated in the Figures, the differences in initial log reduction between the enzymatic cleaner and disinfecting solution combination and the disinfecting solution control are slight, whereas the difference in log reduction after about 2 hours becomes greater for all of the organisms tested, with the exception of C. albicans and A. fumigatus. By comparing the areas under the curves, it is possible to see that the combination of enzyme and disinfecting solution has greater antimicrobial activity than the comparison of D-values would indicate.

### EXAMPLE 4

Approximately 10 ml of the disinfecting solution of Example 1 is placed in an appropriate container. A contact lens to be cleaned is first cleaned with a daily cleaner, such as a surfactant-containing formulation, rinsed with saline, then added to the disinfective solution. This is followed by the addition of one 50 mg tablet of the enzymatic cleaner of Example 1, which will easily dissolve in the disinfecting solution (about 3 min). The lens is allowed to soak in the resultant solution for a period sufficient to clean and disinfect the lens, typically four to eight hours, or overnight. The lens is then taken out and rinsed with saline to remove the soaking solution.

The procedure utilized in connection with embodiments of the present invention which utilize means for delaying release of the enzyme in a solution containing the antimicrobial agent will be similar to that described above, except that the lens to be treated can be added to the solution concurrently with the enzyme, if desired.

The procedure described above may be used with the formulation of Example 2, with a few modifications. Instead of the disinfecting solution of Example 1, a saline solution or other suitable diluent (e.g. distilled water) is used. The tablet of Example 2, containing both the antimicrobial agent and enzyme is dissolved in the saline or other diluent, and the lens to be treated is soaked in the resulting solution. The lens may be added to the solution either prior to or concurrently with the addition of the tablet.

The invention in its broader aspects is not limited to the specific details shown and described above. Departures may be made from such details within the scope of the accompanying claims without departing from the principles of the invention and without sacrificing its advantages.

## Claims

1. A composition for cleaning and disinfecting a contact lens, comprising:
a first component, comprising: an amount of a polymeric antimicrobial agent effective to disinfect the lens, said polymeric antimicrobial agent comprising a nitrogen-containing polymer or copolymer; and an amount of a complexing agent sufficient to provide a concentration of about 0.03 to about 0.7 percent by weight/volume when said composition is dissolved in a solution, said complexing agent being selected from citric acid, ethylenediamthetetraacetic acid, acetic acid and pharmaceutically acceptable salts thereof; and
a second component, comprising an amount of an ophthalmically acceptable enzyme sufficient to provide a concentration of less than or equal to about 26 proteolytic activity units per ml of solution when said composition is dissolved in a solution (wherein a "proteolytic activity unit" is defined as the amount of enzyme activity present to generate one microgram (µg) of tyrosine per minute (µg Tyr/min) as determined by the casein-digestion, colorimetric assay);
wherein said first component and said second component are kept separate from each other prior to use.

2. A composition according to claim 1 , wherein said polymeric antimicrobial agent is selected from polymeric quaternary ammonium compounds, polymeric biguanides, and pharmaceutically acceptable salts thereof.

3. A composition according to claim 2, wherein said polymeric antimicrobial agent is selected from
a) polymeric quaternary ammonium compounds having a number average molecular weight in the range of about 2,000 to about 30,000, said polymeric quaternary ammonium compounds having the formula: 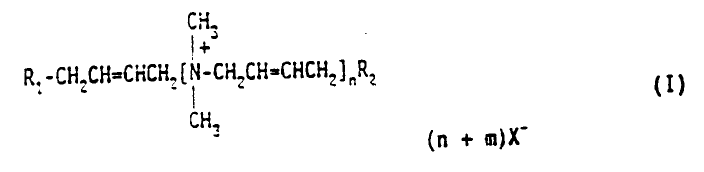 wherein: R_{1,} R₂ = OH, N(CH₃)₂, or N⁺(CH₂CH₂OH)₃; X is a pharmaceutically acceptable anion; and m = 0, 1 or 2; and
b) polymeric biguanides of formula: 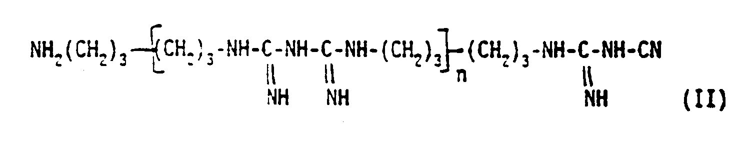 and pharmaceutically acceptable salts thereof, having a number average molecular weight in the range of about 500 to about 120,000.

4. A composition according to claim 3, wherein the concentration of said polymeric antimicrobial agent when the composition is in solution form is in the range of about 0.000001 to about 0.01 percent by weight/volume.

5. A composition according to claim 1 , wherein the polymeric antimicrobial agent is:
α-4-[1-tris (2-hydroxyethyl)ammonium-2-butenyl]poly[1-dimethylammonium-2-butenyl]-ω-tris(2-hydroxyethyl)ammonium chloride (generally known as polyquaternium-1).

6. A composition according to claim 5, wherein the concentration of said polyquaternium-1 is sufficient to provide a concentration of about 0.001% by weight/volume when the composition is in solution form.

7. A composition according to any of claims 1 to 4, wherein said polymeric antimicrobial agent comprises a mixture of the polymeric quaternary ammonium compounds of Formula (I).

8. A composition according to any of claims 1 to 7, wherein said enzyme is selected from pancreatin, papain, and subtilisin.

9. A composition according to any of claims 5, 6, and 8, wherein the polymeric antimicrobial agent is polyquaternium-1 and the enzyme is pancreatin.

10. A composition according to any of claims 1 to 4 and 8, wherein the polymeric antimicrobial agent is a biguanide polymer of Formula (II) as defined in claim 3 or pharmaceutically acceptable salt thereof having a number average molecular weight in the range of about 500 to 2,500.

11. A composition according to claim 10, wherein the concentration of said biguanide polymer II is sufficient to provide a concentration of about 0.00005% by weight/volume when the composition is in solution form.

12. A composition according to any of claims 1 to 11, wherein the ionic strength of the composition when in solution form is maintained at an isotonic or hypotonic level.

13. A composition according to claim 12. wherein the ionic strength of the composition when in solution form is in the range of about 150 to about 300 mOsm/kg.

14. A composition according to any of claims 1 to 13, wherein the concentration of said enzyme when the composition is in solution form is less than or equal to about 13 "proteolytic activity units" (as defined in claim 1) per ml of solution.

15. A composition according to any of claims 1 to 14, wherein said second component further comprises an ophthalmically acceptable enzyme having lipolytic mucolytic or amylolytic activity.

16. A composition according to any of claims 1 to 15, wherein said second component is formulated as a tablet.

17. A composition according to claim 16, wherein said tablet comprises an enzyme core and an outer coating to delay release of said enzyme core when said tablet is added to said first component.

18. A method for simultaneously cleaning and disinfecting a contact lens, comprising the steps of:
immersing the contact lens in an aqueous solution comprising: an amount of a polymeric antimicrobial agent effective to disinfect the contact lens, said polymeric antimicrobial agent comprising a nitrogen-containing polymer or co-polymer; and about 0.03 to 0.7 percent by weight/volume of a complexing agent, said complexing agent being selected from citric acid, ethylenediaminetetraacetic acid, acetic acid and pharmaceutically acceptable salts thereof;
releasing into said aqueous solution an amount of an ophthalmically acceptable enzyme sufficient to provide a concentration of less than or equal to about 26 "proteolytic activity units" (as defined in claim 1); and
maintaining immersion of the contact lens in said aqueous solution for a period sufficient to clean and disinfect the lens.

19. A method according to claim 18, wherein the polymeric antimicrobial agent is selected from polymeric quaternary ammonium compounds, polymeric biguanides and pharmaceutically acceptable salts thereof.

20. A method according to claim 19, wherein the polymeric antimicrobial agent is selected from:
a) polymeric quaternary ammonium compounds having a number average molecular weight in the range of about 2,000 to about 30,000, said polymeric quaternary ammonium compounds having the formula: 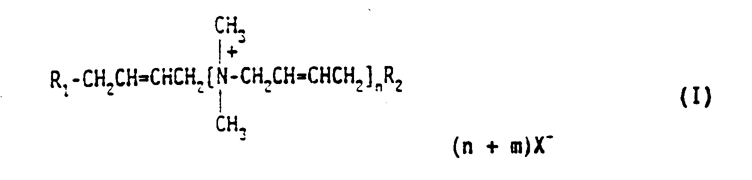 wherein: R₁ R₂ = OH, N(CH₃)₂, or N ⁺(CH₂CH₂OH)₃; X is a pharmaceutically acceptable anion; and m = 0, 1 or 2; and b) polymeric biguanides of formula: 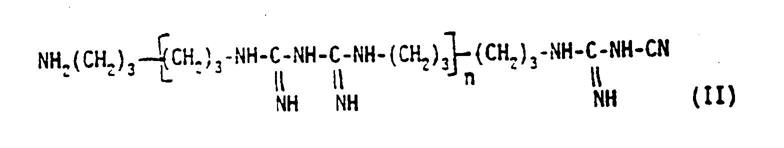 and pharmaceutically acceptable salts thereof, having a number average molecular weight in the range of about 500 to about 120,000.

21. A method according to claim 20, wherein the concentration of said polymeric antimicrobial agents when the composition is in solution form is in the range of about 0.000001% to about 0.01% by weight/volume.

22. A method according to claim 18, wherein the polymeric antimicrobial agent is:
α-4-[1-tris(2-hydroxyethyl)ammonium-2-butenyl]poly[1-dimethylamrnonium-2-butenyl]-ω-tris(2-hydroxyethyl)ammonium chloride (generally known as polyquaternium-1).

23. A method according to claim 18, wherein the polymeric antimicrobial agent is a biguanide polymer of Formula (II) as defined in claim 3 or a pharmaceutically acceptable salt thereof having a number average molecular weight in the range of about 500 to 2,500.

24. A method for simultaneously cleaning and disinfecting a contact lens according to claim 18, wherein the antimicrobial agent is:
a) about 0.001 wt% of the polymer defined in claim 5; or
b) about 0.00005 wt% of the polymer defined in claim 10; and
the enzyme is selected from pancreatin and subtilisin.

25. A method according to any of claims 18 to 24, wherein the concentration of said enzyme is less than or equal to about 13 "proteolytic activity units" (as defined in claim 1) per ml of solution.

26. A method according to any of claims 18 to 25, wherein the ionic strength of the aqueous system is maintained at an isotonic or hypotonic level.

27. A method according to claim 26, wherein the ionic strength of the aqueous system is in the range of from about 150 to about 300 mOsm/kg.

28. A method according to claim 24, wherein the proteolytic enzyme is contained in an enzymatic cleaning composition, and said enzymatic cleaning composition is formulated as a tablet comprising approximately 1.38 mg of pancreatin (6X), 5.95 mg of citric acid. 13.135 mg of sodium bicarbonate. 0.415 mg of povidone. 0.75 mg of polyethylene glycol (3350) and an amount of compressible sugar sufficient to make a total weight of 50 mg.

29. A method according to claim 18, wherein the lens is soaked in the aqueous system overnight.

30. A method according to claim 18, wherein the lens is soaked in the aqueous system for four to eight hours .

31. A method according to any one of claims 18 to 30, wherein said proteolytic enzyme is surrounded by a delayed release coating to delay release of said enzyme in said aqueous system by up to two hours.

32. A method of simultaneously cleaning and disinfecting a contact lens according to claim 18, which comprises:
placing 10 ml of a disinfecting solution in a container, said disinfecting solution containing 0.001 + 10% excess w/v% polyquaternium-1. (as defined in claim 5), 0.48 w/v% sodium chloride. 0.05 w/v% disodium edetate, 0.021 w/v% citric acid monohydrate, 0.56 w/v% sodium citrate dihydrate and an amount of purified water sufficient to make a final volume of 10 ml:
placing the lens in said disinfecting solution:
dissolving a 50 mg enzymatic cleaner tablet in said disinfecting solution to form a disinfectant/enzyme solution, said tablet containing 1 .38 mg of pancreatin (6X), 5.95 mg of citric acid. 13.135 mg of sodium bicarbonate. 0.415 mg of povidone. 0.75 mg of polyethylene glycol (3350) and an amount of compressible sugar sufficient to make a total weight of 50 mg; and
soaking the lens in the disinfectant/enzyme solution for a period sufficient to clean and disinfect the lens.

## Patentansprüche

1. Mittel zur Reinigung und Desinfektion von Kontaktlinsen, enthaltend:
eine erste Komponente, umfassend eine Menge eines zur Desinfektion der Linsen wirksamen polymeren antimikrobiellen Mittels, wobei das polymere antimikrobielle Mittel ein Stickstoff enthaltendes Polymeres oder Copolymeres umfaßt, und eine Menge eines Komplexbildners, die ausreichend ist, eine Konzentration von etwa 0,03 bis etwa 0,7 Gew./Vol.-% zu ergeben, wenn das Mittel in einer Lösung aufgelöst ist, wobei der Komplexbildner aus Citronensäure, Ethylendiamintetraessigsäure, Essigsäure und pharmazeutisch annehmbaren Salzen davon ausgewählt ist; und
eine zweite Komponente, umfassend eine Menge eines ophthalmologisch annehmbaren Enzyms, die ausreichend ist, eine Konzentration von etwa 26 proteolytischen Aktivitätseinheiten pro ml Lösung oder weniger zu ergeben, wenn das Mittel in einer Lösung aufgelöst ist (wobei eine "proteolytische Aktivitätseinheit" als die Menge der vorhandenen Enzymaktivität zur Erzeugung von einem Mikrogramm (µg) Tyrosin pro Minute (µg Tyr/min), bestimmt durch einen kolorimetrischen Casein-Digestionsassay, definiert ist);
wobei die genannte erste Komponente und die genannte zweite Komponente vor dem Gebrauch voneinander getrennt gehalten werden.

2. Mittel nach Anspruch 1, dadurch **gekennzeichnet,** daß das polymere antimikrobielle Mittel aus polymeren quaternären Ammoniumverbindungen, polymeren Biguaniden und pharmazeutisch annehmbaren Salzen davon ausgewählt ist.

3. Mittel nach Anspruch 2, dadurch **gekennzeichnet**, daß das polymere antimikrobielle Mittel aus
a) polymeren quaternären Ammoniumverbindungen mit einem zahlenmittleren Molekulargewicht im Bereich von etwa 2000 bis etwa 30.000, wobei die polymeren quaternären Ammoniumverbindungen die Formel: 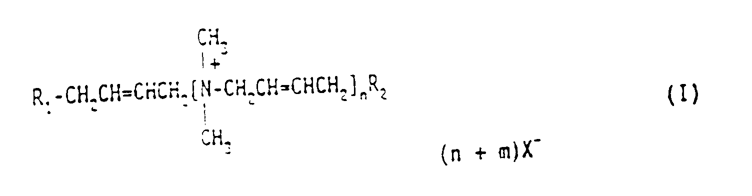 haben, worin: R₁, R₂ = OH, N(CH₃)₂ oder N⁺(CH₂CH₂OH)₃; X für ein pharmazeutisch annehmbares Anion steht; und m = 0, 1 oder 2; und
b) polymeren Biguaniden der Formel: 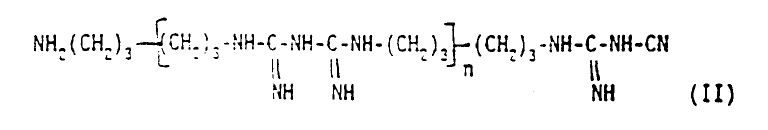 und pharmazeutisch annehmbaren Salzen davon mit einem zahlenmittleren Molekulargewicht im Bereich von etwa 500 bis etwa 120.000
ausgewählt ist.

4. Mittel nach Anspruch 3, dadurch **gekennzeichnet**, daß die Konzentration des polymeren antimikrobiellen Mittels, wenn das Mittel in Lösungsform vorliegt, im Bereich von etwa 0,000001 bis etwa 0,01 Gew./Vol.-% liegt.

5. Mittel nach Anspruch 1, dadurch **gekennzeichnet**, daß das polymere antimikrobielle Mittel
α-4-[1-Tris(2-hydroxyethyl)ammonium-2-butenyl]poly[1-dimethylammonium-2-butenyl]-ω-tris(2-hydroxyethyl) - ammoniumchlorid (allgemein als Polyquaternium-1 bekannt)
ist.

6. Mittel nach Anspruch 5, dadurch **gekennzeichnet**, daß die Konzentration von Polyquaternium-1 ausreichend ist, um eine Konzentration von etwa 0,001 Gew./Vol.-% zu ergeben, wenn das Mittel in Lösungsform vorliegt.

7. Mittel nach einem der Ansprüche 1 bis 4, dadurch **gekennzeichnet,** daß das polymere antimikrobielle Mittel ein Gemisch aus polymeren quaternären Ammoniumverbindungen der Formel (I) umfaßt.

8. Mittel nach einem der Ansprüche 1 bis 7, dadurch **gekennzeichnet**, daß das Enzym aus Pancreatin, Papain und Subtilisin ausgewählt ist.

9. Mittel nach einem der Ansprüche 5, 6 und 8, dadurch **gekennzeichnet,** daß das polymere antimikrobielle Mittel Polyquaternium-1 ist und daß das Enzym Pancreatin ist.

10. Mittel nach einem der Ansprüche 1 bis 4 und 8, dadurch **gekennzeichnet,** daß das polymere antimikrobielle Mittel ein Biguanidpolymeres der Formel (II), wie in Anspruch 3 definiert, oder ein pharmazeutisch annehmbares Salz davon mit einem zahlenmittleren Molekulargewicht im Bereich von etwa 500 bis 2.500 ist.

11. Mittel nach Anspruch 10, dadurch **gekennzeichnet,** daß die Konzentration des genannten Biguanidpolymeren II ausreichend ist, um eine Konzentration von etwa 0,00005 Gew./Vol.-%, wenn das Mittel in Lösungsform vorliegt, zu ergeben.

12. Mittel nach einem der Ansprüche 1 bis 11, dadurch **gekennzeichnet,** daß die Ionenstärke des Mittels, wenn es in Lösungsform vorliegt, auf einem isotonischen oder hypotonischen Niveau gehalten wird.

13. Mittel nach Anspruch 12, dadurch **gekennzeichnet,** daß die Ionenstärke des Mittels, wenn es in Lösungsform vorliegt, im Bereich von etwa 150 bis etwa 300 mOsm/kg liegt.

14. Mittel nach einem der Ansprüche 1 bis 13, dadurch **gekennzeichnet,** daß die Konzentration des genannten Enzyms, wenn das Mittel in Lösungsform vorliegt, etwa 13 "proteolytischen Aktivitätseinheiten" (wie in Anspruch 1 definiert) pro ml Lösung gleich ist oder kleiner ist.

15. Mittel nach einem der Ansprüche 1 bis 14, dadurch **gekennzeichnet,** daß die genannte zweite Komponente weiterhin ein ophthalmologisch annehmbares Enzym mit einer lipolytischen, mucolytischen oder amylolytischen Aktivität umfaßt.

16. Mittel nach einem der Ansprüche 1 bis 15, dadurch **gekennzeichnet,** daß die zweite Komponente als Tablette formuliert ist.

17. Mittel nach Anspruch 16, dadurch **gekennzeichnet,** daß die Tablette einen Enzymkern und einen Außenüberzug umfaßt, um die Freisetzung des Enzymkerns zu verzögern, wenn die genannte Enzymtablette zu der genannten ersten Komponente gegeben wird.

18. Verfahren zur gleichzeitigen Reinigung und Desinfektion von Kontaktlinsen, umfassend die Stufen:
Eintauchen der Kontaktlinsen in eine wäßrige Lösung, die eine wirksame Menge eines polymeren antimikrobiellen Mittels zur Desinfektion der Kontaktlinsen, wobei das genannte polymere antimikrobielle Mittel ein Stickstoff enthaltendes Polymeres oder Copolymeres umfaßt, und etwa 0,03 bis 0,7 Gew./Vol.-% eines Komplexbildners, wobei der Komplexbildner aus Citronensäure, Ethylendiamintetraessigsäure, Essigsäure und pharmazeutisch annehmbaren Salzen davon ausgewählt ist, enthält;
Freisetzen in die genannte wäßrige Lösung einer Menge eines ophthalmologisch annehmbaren Enzyms, die ausreichend ist, um eine Konzentration von etwa 26 "proteolytischen Aktivitätseinheiten" (wie in Anspruch 1 definiert) oder weniger zu ergeben, und
Aufrechterhalten des Eintauchens der Kontaktlinsen in die genannte wäßrige Lösung über einen genügenden Zeitraum zur Reinigung und Desinfektion der Linsen.

19. Verfahren nach Anspruch 18, dadurch **gekennzeichnet,** daß das polymere antimikrobielle Mittel aus polymeren quaternären Ammoniumverbindungen, polymeren Biguaniden und pharmazeutisch annehmbaren Salzen davon ausgewählt ist.

20. Verfahren nach Anspruch 19, dadurch **gekennzeichnet,** daß das polymere antimikrobielle Mittel aus
a) polymeren quaternären Ammoniumverbindungen mit einem zahlenmittleren Molekulargewicht im Bereich von etwa 2000 bis etwa 30.000, wobei die polymeren quaternären Ammoniumverbindungen die Formel: 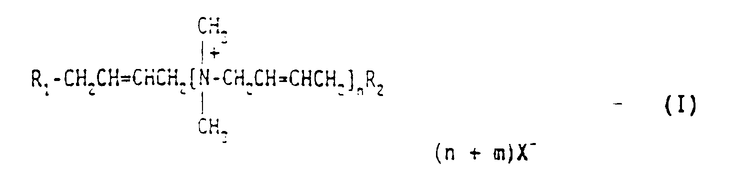 haben, worin: R₁, R₂ = OH, N(CH₃)₂ oder N⁺(CH₂CH₂OH)₃; X für ein pharmazeutisch annehmbares Anion steht; und m = 0, 1 oder 2; und
b) polymeren Biguaniden der Formel: 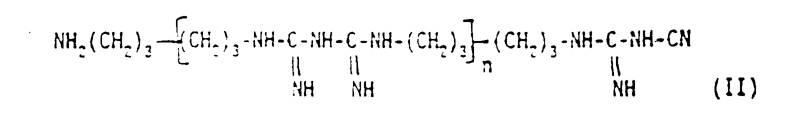 und pharmazeutisch annehmbaren Salzen davon mit einem zahlenmittleren Molekulargewicht im Bereich von etwa 500 bis etwa 120.000
ausgewählt ist.

21. Verfahren nach Anspruch 20, dadurch **gekennzeichnet,** daß die Konzentration des polymeren antimikrobiellen Mittels, wenn das Mittel in Lösungsform vorliegt, im Bereich von etwa 0,000001 bis etwa 0,01 Gew./Vol.-% liegt.

22. Verfahren nach Anspruch 18, dadurch **gekennzeichnet,** daß das polymere antimikrobielle Mittel
α-4-[1-Tris(2-hydroxyethyl)ammonium-2-butenyl]poly[1-dimethylammonium-2-butenyl]-ω-tris(2-hydroxyethyl)-ammoniumchlorid (allgemein als Polyquaternium-1 bekannt)
ist.

23. Verfahren nach Anspruch 18, dadurch **gekennzeichnet,** daß das polymere antimikrobielle Mittel ein Biguanidpolymeres der Formel (II), wie in Anspruch 3 definiert, oder ein pharmazeutisch annehmbares Salz davon mit einem zahlenmittleren Molekulargewicht im Bereich von etwa 500 bis 2.500 ist.

24. Verfahren zur gleichzeitigen Reinigung und Desinfektion von Kontaktlinsen nach Anspruch 18, dadurch **gekennzeichnet**, daß das antimikrobielle Mittel
a) etwa 0,001 Gew.-% des in Anspruch 5 definierten Polymeren; oder
b) etwa 0,00005 Gew.-% des in Anspruch 10 definierten Polymeren
ist und daß das Enzym aus Pancreatin und Subtilisin ausgewählt ist.

25. Verfahren nach einem der Ansprüche 18 bis 24, dadurch **gekennzeichnet,** daß die Konzentration des genannten Enzyms, wenn das Mittel in Lösungsform vorliegt, etwa 13 "proteolytischen Aktivitätseinheiten" (wie in Anspruch 1 definiert) pro ml Lösung gleich ist oder kleiner ist.

26. Verfahren nach einem der Ansprüche 18 bis 25, dadurch **gekennzeichnet,** daß die Ionenstärke des wäßrigen Systems auf einem isotonischen oder hypotonischen Niveau gehalten wird.

27. Verfahren nach Anspruch 26, dadurch **gekennzeichnet,** daß die Ionenstärke des wäßrigen Systems im Bereich von etwa 150 bis etwa 300 mOsm/kg liegt.

28. Verfahren nach Anspruch 24, dadurch **gekennzeichnet,** daß das proteolytische Enzym in einem enzymatischen Reinigungsmittel enthalten ist und daß das enzymatische Reinigungsmittel als Tablette formuliert ist, die ungefähr 1,38 mg Pancreatin (6X), 5,95 mg Citronensäure, 13,135 mg Natriumbicarbonat, 0,415 mg Povidon, 0,75 mg Polyethylenglykol (3350) und eine ausreichende Menge von komprimierbarem Zucker, daß ein Gesamtgewicht von 50 mg erhalten wird, enthält.

29. Verfahren nach Anspruch 18, dadurch **gekennzeichnet,** daß die Linsen über Nacht in dem wäßrigen System eingeweicht wird.

30. Verfahren nach Anspruch 18, dadurch **gekennzeichnet,** daß die Linsen in dem wäßrigen System vier bis acht Stunden eingeweicht werden.

31. Verfahren nach einem der Ansprüche 18 bis 30, dadurch **gekennzeichnet,** daß das proteolytische Enzym von einem Überzug zur verzögerten Freisetzung umgeben ist, um die Freisetzung des Enzyms in dem wäßrigen System bis zu zwei Stunden zu verzögern.

32. Verfahren zur gleichzeitigen Reinigung und Desinfektion von Kontaktlinsen nach Anspruch 18, umfassend:
Einbringung von 10 ml einer Desinfektionslösung in einen Behälter, wobei die Desinfektionslösung 0,001 + 10% Überschuß Gew./Vol.-% Polyquaternium-1 (wie in Anspruch 5 definiert), 0,48 Gew./Vol.-% Natriumchlorid, 0,05 Gew./Vol.-% Dinatriumedetat, 0,021 Gew./Vol.-% Citronensäuremonohydrat, 0,56 Gew./Vol.-% Natriumcitratdihydrat und eine ausreichende Menge von gereinigtem Wasser, um ein Endvolumen von 10 ml zu erhalten, enthält;
Einbringen der Linsen in die genannte Desinfektionslösung;
Auflösen einer 50 mg-Tablette eines enzymatischen Reinigers in der genannten Desinfektionslösung, um eine Desinfektions/Enzymlösung zu bilden, wobei die genannte Tablette 1,38 mg Pancreatin (6X), 5,95 mg Citronensäure, 13,135 mg Natriumbicarbonat, 0,415 mg Povidon, 0,75 mg Polyethylenglykol (3350) und eine ausreichende Menge von komprimierbarem Zucker, daß ein Gesamtgewicht von 50 mg erhalten wird, enthält; und
Einweichen der Linsen in der Desinfektions/Enzymlösung über einen ausreichenden Zeitraum zur Reinigung und Desinfektion der Linsen.

## Revendications

1. Composition pour nettoyer et désinfecter une lentille de contact, comprenant:
un premier composant comprenant une quantité d'un agent polymère antimicrobien efficace pour désinfecter la lentille, ledit agent polymère antimicrobien comprenant un polymère ou copolymère azoté et une certaine quantité d'un agent complexant suffisant pour obtenir une concentration d'environ 0,03 à environ 0,7% en poids/volume quand ladite composition est dissoute dans une solution, ledit agent complexant étant choisi parmi l'acide citrique, l'acide éthylènediaminetétracétique, l'acide acétique et les sels pharmaceutiquement acceptables de ces acides; et
un deuxième composant comprenant une quantité d'une enzyme ophthalmiquement acceptable, suffisante pour obtenir une concentration égale ou inférieure à environ 26 unités d'activité protéolytique par ml de solution quand le composition est dissoute dans une solution (l'unité d'activité protéolytique étant définie comme la quantité d'activité enzymatique présente pour créer un microgramme (µg) de tyrosine par minute (µg Tyr/min) comme déterminé par l'essai colorimétrique de digestion de la caséine;
dans laquelle le premier composant et le deuxième composant sont maintenus séparés l'un de l'autre avant l'utilisation.

2. Composition selon la revendication 1, dans laquelle ledit agent polymère antimicrobien est choisi parmi les composés polymères de l'ammonium quaternaire, les biguanides polymère et les sels pharmaceutiquement acceptables de ceux-ci.

3. Composition selon la revendication 2, dans laquelle ledit agent polymère antimicrobien est choisi parmi:
a) les composés polymères de l'ammonium quaternaire ayant un poids moléculaire moyen en nombre compris dans l'intervalle allant de 2.000 à environ 30.000, lesdits composés polymères de l'ammonium quaternaire ayant pour formule 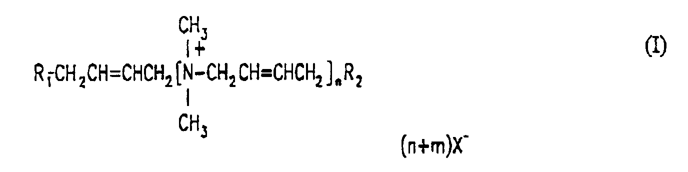 dans laquelle R₁, R₂ = OH, N(CH₃)₂, ou N⁺(CH₂CH₂OH)₃; X est un anion pharmaceutiquement acceptable; et m = 0, 1 ou 2; et
b) les biguanides polymères de formule: 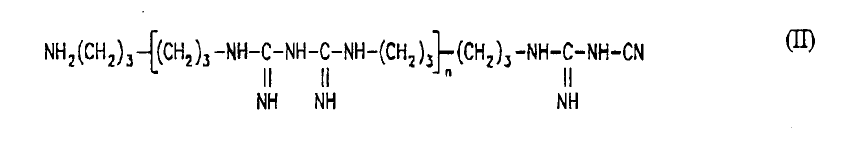 et les sels pharmaceutiquement acceptable de ceux-ci, ayant un poids moléculaire moyen en nombre compris dans l'intervalle allant d'environ 500 à environ 120.000.

4. Composition selon la revendication 3, dans laquelle la concentration dudit agent polymère antimicrobien, quand la composition est sous forme de solution, est comprise dans l'intervalle allant d'environ 0,000001 à environ 0,01% en poids/volume.

5. Composition selon la revendication 1, dans laquelle l'agent polymère antimicrobien est
le chlorure de α-4-[1-tris(2-hydroxyéthyl)ammonium-2-butényl]poly[1-diméthylammonium-2-butényl]-ω-tris(2-hydroxéthyl)ammonium (appelé généralement polyquaternium-1).

6. Composition selon la revendication 5, dans laquelle la concentration dudit polyquaternium-1 est suffisante pour obtenir une concentration d'environ 0,001% en poids/volume quand la composition est sous forme de solution.

7. Composition selon l'une quelconque des revendications 1 à 4, dans laquelle ledit agent polymère antimicrobien comprend un mélange de composés polymères de l'ammonium quaternaire répondant à la formule (I).

8. Composition selon l'une quelconque des revendications 1 à 7, dans laquelle ledit enzyme est choisi parmi la pancréatine, la papaïne et la subtilisine.

9. Composition selon l'une quelconque des revendications 5, 6 et 8, dans laquelle l'agent polymère antimicrobien est le polyquaternium-1 et l'enzyme est la pancréatine.

10. Composition selon l'une quelconque des revendications 1 à 4 et 8, dans laquelle l'agent polymère antimicrobien est un biguanide polymère de formule (II) tel que défini par la revendication 3 ou les sels pharmaceutiquement acceptables de celui-ci ayant un poids moléculaire moyen en nombre compris dans l'intervalle allant d'environ 500 à 2.500.

11. Composition selon la revendication 10, dans laquelle la concentration du biguamide polymère II est suffisante pour obtenir une concentration d'environ 0,00005% en poids/volume quand la composition est sous forme de solution.

12. Composition selon l'une quelconque des revendications 1 à 11, dans laquelle la force ionique de la composition quand la forme de solution est maintenue à une valeur isotonique ou hypotonique.

13. Composition selon la revendication 12, dans laquelle la force ionique de la composition, quand la composition est sous forme de solution, est comprise dans l'intervalle d'environ 150 à environ 300 mOsm/kg.

14. Composition selon l'une quelconque des revendications 1 à 13, dans laquelle la concentration de ladite enzyme, quand la composition est sous forme de solution, est inférieure ou égale à environ 13 "unités d'activité protéolyique" (telles que définies par la revendication 1) par ml de solution.

15. Composition selon l'une quelconque des revendications 1 à 14, dans laquelle ledit deuxième composant comprend en outre une enzyme ophthalmiquement acceptable ayant une activité lipolytique, mucolytique ou amylolytique.

16. Composition selon l'une quelconque des revendications 1 à 15, dans laquelle ledit deuxième composant est formulé sous forme de comprimés.

17. Composition selon la revendication 16, dans laquelle ledit comprimé comprend un noyau d'enzyme et un revêtement extérieur pour retarder le dégagement dudit noyau d'enzyme quand ledit comprimé est ajouté audit premier composant.

18. Procédé pour nettoyer et désinfecter simultanément une lentille de contact comprenant les opérations suivantes:
immerger la lentille de contact dans une solution aqueuse comprenant: une quantité d'agent polymère antimicrobien efficace pour désinfecter la lentille de contact, ledit agent polymère antimicrobien comprenant un polymère ou copolymère azoté et environ de 0,03 à 0,7% en poids/volume d'un agent complexant, ledit agent complexant étant choisi parmi l'acide citrique, l'acide éthylènediaminetétraacétique, l'acide acétique et les sels pharmaceutiquement acceptables de ces acides;
dégager dans ladite solution aqueuse une quantité d'une enzyme ophthalmiquement acceptable, suffisante pour obtenir une concentration égale ou inférieure à environ 26 unités d'activité protéolytique (telles que définies par la revendication 1); et
maintenir l'immersion de la lentille de contact dans ladite solution aqueuse pendant une période suffisante pour nettoyer et désinfecter la lentille.

19. Procédé selon la revendication 18, dans lequel l'agent polymère antimicrobien est choisi parmi les composés polymères de l'ammonium quaternaire, les biguanides polymères et les sels pharmaceutiquement acceptables de ceux-ci.

20. Procédé selon la revendication 19, dans lequel un agent polymère antimicrobien est choisi parmi :
a) les composés polymères de l'ammonium quaternaire ayant un poids moléculaire moyen en nombre compris dans l'intervalle allant d'environ 2.000 à environ 30.000, lesdits composés polymères de l'ammonium quaternaire ayant pour formule: 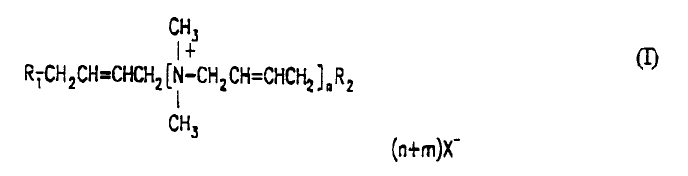 dans laquelle R₁, R₂ = OH, N(CH₃)₂, ou N⁺(CH₂CH₂OH)₃; X est un anion pharmaceutiquement acceptable; et m = 0, 1 ou 2; et
b) les biguanides polymères de formule: 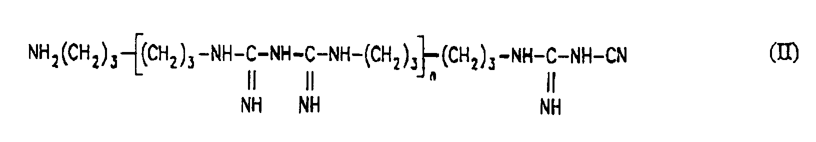 et les sels pharmaceutiquement acceptable de ceux-ci, ayant un poids moléculaire moyen en nombre compris dans l'intervalle allant d'environ 500 à environ 120.000.

21. Procédé selon la revendication 20, dans lequel la concentration desdits agents polymères antimicrobiens, quand la composition est sous forme de solution, est comprise dans l'intervalle allant d'environ 0,000001 à environ 0,01% en poids/volume.

22. Procédé selon la revendication 18, dans lequel l'agent polymère antimicrobien est
le chlorure de α-4-[1-tris(2-hydroxyéthyl)ammonium-2-butényl]poly[1-diméthylamonium-2-butényl]-ω-tris(2-hydroxéthyl)ammonium (appelé généralement polyquaternium-1).

23. Procédé selon la revendication 18, dans lequel l'agent polymère antimicrobien est un polymère de biguanide de formule (II) tel que défini par la revendication 3 ou un sel pharmaceutiquement acceptables de celui-ci ayant un poids moléculaire moyen en nombre compris dans l'intervalle allant d'environ 500 à 2.500.

24. Procédé pour nettoyer et désinfecter simultanément une lentille de contact selon la revendication 18, dans lequel l'agent antimicrobien est:
a) environ 0,001% en poids du polymère défini par revendication 5; ou
b) environ 0,00005% en poids du polymère défini par la revendication 10; et
l'enzyme est choisie parmi la pancréatine et la subtilisine. olume quand la composition est sous forme de solution.

25. Procédé selon l'une quelconque des revendications 18 à 24, dans lequel la concentration de ladite enzyme est inférieure ou égale à environ 13 "unités d'activité protéolyique" (telles que définies par la revendication 1) par ml de solution.

26. Procédé selon l'une quelconque des revendications 18 à 25, dans lequel la force ionique du système aqueux est maintenue à une valeur isotonique ou hypotonique.

27. Procédé selon la revendication 26, dans lequel la force ionique du système aqueux est comprise dans l'intervalle allant d'environ 150 à environ 300 mOsm/kg.

28. Procédé selon la revendication 24, dans lequel l'enzyme protéolytique est présente dans une composition enzymatique de nettoyage et ladite composition enzymatique de nettoyage est formulée sous forme ce comprimés comprenant approximativement 1,38 mg de pancréatine (6X), 5,95 mg d'acide citrique, 13,135 mg de bicarbonate de sodium, 0,415 mg de povidone, 0,75 mg de polyéthylène-glycol (3350) et une quantité de sucre compressible suffisante pour obtenir un poids total de 50 mg.

29. Procédé selon la revendication 18, dans lequel la lentille est immergée dans le système aqueux pendant une nuit.

30. Procédé selon la revendication 18, dans lequel la lentille est immergée dans le système aqueux pendant 4 à 8 heures.

31. Procédé selon l'une quelconque des revendication 18 à 30, dans lequel ladite enzyme protéolytique est entourée par un revêtement permettant un dégagement retardé afin de retarder le dégagement de ladite enzyme dans ledit système aqueux pendant une période allant jusque 2 heures.

32. Procédé pour nettoyer et désinfecter simultanément une lentille de contact, selon la revendication 18, qui comprend:
le placement de 10 ml de solution désinfectante dans un récipient, ladite solution désinfectant contenant de 0,001 + 10% en excès poids/volume de polyquaternium-1 (tel que défini par la revendication 5), 0,48% poids/volume de chlorure de sodium, 0,05% poids/volume d'édétate disodique, 0,021% poids/volume d'acide citrique monohydraté, 0,56% poids/volume de citrate sodique dihydraté et une quantité d'eau purifiée suffisante pour obtenir un volume final de 10 ml;
placer la lentille dans ladite solution désinfectante;
dissoudre un comprimé de produit de nettoyage enzymatique de 50 mg dans ladite solution désinfectante pour former une solution enzymatique désinfectante, ledit comprimé contenant 1,38 mg de pancréatine (6X), 5,95 mg d'acide citrique, 13,135 mg de bicarbonate de sodium, 0,415 mg de povidone, 0,75 mg de polyéthylène-glycol (3350) et une quantité de sucre compressible suffisante pour obtenir un poids total de 50 mg; et
immerger la lentille dans la solution enzymatique désinfectante pendant une période suffisante pour nettoyer et désinfecter la lentille.
